(19) **Europäisches Patentamt**

**European Patent Office**

**Office européen des brevets**

(11) **EP 1 062 974 B1**

(12) **EUROPÄISCHE PATENTSCHRIFT**

(45) Veröffentlichungstag und Bekanntmachung des Hinweises auf die Patenterteilung:
**25.08.2004 Patentblatt 2004/35**

(51) Int Cl.⁷: **A61N 1/365**

(21) Anmeldenummer: **00112632.5**

(22) Anmeldetag: **15.06.2000**

(54) **Verfahren zur Bestimmung eines variablen Steuer-Parameters eines medizinischen Geräteimplantates**

Method of determining of a variable control parameter of an implantable medical device

Procédé de détermination d'un paramètre de commande variable d'un appareil médical implantable

(84) Benannte Vertragsstaaten:
**AT BE CH CY DE DK ES FI FR GB GR IE IT LI LU MC NL PT SE**

(30) Priorität: **23.06.1999 DE 19928659**

(43) Veröffentlichungstag der Anmeldung:
**27.12.2000 Patentblatt 2000/52**

(73) Patentinhaber: BIOTRONIK Mess- und Therapiegeräte GmbH & Co Ingenieurbüro Berlin
**12359 Berlin (DE)**

(72) Erfinder:
• **Graupner, Harald, Dr.**
**91056 Erlangen (DE)**
• **Lippert, Michael, Dr.**
**91522 Ansbach (DE)**

(74) Vertreter: **Hübner, Gerd, Dipl.-Phys. et al Rau, Schneck & Hübner Patentanwälte Königstrasse 2 90402 Nürnberg (DE)**

(56) Entgegenhaltungen:
**EP-A- 0 793 976          US-A- 5 645 575**

• **URBASZEK, A., PICHLMAIER, A. M., SCHALDACH, M., HUTTEN, H.: "Intrakardiale Impedanzmessung zur Bestimmung der Sympathikusaktivität bei frequenzadaptiver Elektrostimulation - Teil 1: Biomedizinische Grundlagen" BIOMEDIZINISCHE TECHNIK, Bd. 37, Nr. 7-8, 1992, Seiten 155-161, XP002225289**

**Beschreibung**

Die Erfindung betrifft ein medizinisches Geräteimplantat mit einem Verfahren zur Bestimmung eines variablen, insbesondere physiologischen Steuer-Parameters, insbesondere zur Herzratenadaption eines Herzschrittmachers.

Wenngleich die Erfindung im Zusammenhang mit verschiedenartigen medizinischen Geräteimplantaten einsetzbar ist, läßt sich der Hintergrund der Erfindung zur besseren Veranschaulichung anhand des Beispiels eines Herzschrittmachers erläutern; wie sie in der EP-A-0 793 976 oder des US-A-5 645 575 offenbart sind. Biomedizinische Grundlagen sind in diesem Zusammenhang dem Fachartikel von Urbaszek, A., Pichlmaier, A. M., Schaldach, M., Hutten, H.: "Intrakardiale Impedanzmessung zur Bestimmung der Sympathikusaktivität bei frequenzadaptiver Elektrostimulation - Teil 1: Biomedizinische Grundlagen", in Biomedizinische Technik, Bd. 37, Nr.7-8, 1992, Seiten 155-161 entnehmbar.

Ein moderner Herzschrittmacher stellt ein medizinisches Geräteimplantat dar, bei dem ein bestimmter physiologischer Steuer-Parameter ermittelt und eingestellt werden muß. Bei diesem Steuer-Parameter handelt es sich beispielsweise um die Herzrate, also die Frequenz, mit der das Herz ggf. durch Aufbringen eines Stimulationsimpulses durch den Herzschrittmacher schlägt. Die Herzrate eines Schrittmacher-gestützten Herzens ist folglich vergleichbar mit der Pulsfrequenz des gesunden Herzens.

Bekanntermaßen ist die Pulsfrequenz und folglich auch die optimale Herzrate, wie sie von einem Herzschrittmacher zu erbringen ist, abhängig vom aktuellen Zustand des Patienten. Treibt dieser beispielsweise Sport, so ist die Herzrate höher einzustellen, als im Ruhezustand des Patienten. Neben der Erhöhung der Herzrate oder Pulsfrequenz wird dabei eine gesteigerte Auswurfleistung des Herzens auch durch eine erhöhte Kontraktilität erzielt. In diesem Zusammenhang ist eine verbreitete Störung des Herzens dadurch gegeben, daß zwar einerseits die natürliche Ratenadaption des Herzens durch den Sinus-Knoten nicht mehr funktioniert, daß aber nach wie vor eine natürliche Adaption des Kontraktionsverhaltens des Herzens an die jeweils aktuellen physiologischen Bedürfnisse des Patienten vorhanden ist. Ein sich so anpassendes Herz ändert sein Kontraktionsverhalten beispielsweise durch Änderung des Schlagvolumens oder der Kontraktionsgeschwindigkeit. Diese Änderung des Kontraktionsverhaltens kann - wie aus dem Stand der Technik bekannt ist - als Ausgangsgröße gemessen werden, wobei das Kontraktionsverhalten für die Herzrate signifikant ist. Als Basis-Parameter für das Kontraktionsverhalten ist nun z. B. die intrakardiale Impedanz durch eine unipolare Messung zu erfassen. Dabei wird zwischen einer sogenannten Tip-Elektrode in der Spitze des Ventrikels und dem Schrittmachergehäuse ein Meß-Strom angelegt, der beispielsweise aus biphasischen Rechteck-Pulsen einer Breite von wenigen zehn Mikrosekunden und einer Folge-Frequenz von einigen zehn bis einigen hundert Hertz besteht. Die Änderung der intrakardialen Impedanz aufgrund einer Kontraktion des Herzens beruht auf dem sich ändernden Verhältnis von hochohmigem Herzmuskelgewebe zu niederohmigem Blut in der Umgebung der Tip-Elektrode. Für ein bestimmtes Kontraktionsverhalten ergibt sich ein repräsentativer Meß-Signalverlauf für die intrakardiale Impedanz als Basis-Parameter. Die intrakardiale Impedanz bildet damit also die Dynamik des Kontraktionsvorganges ab.

Diese gemessene Dynamik des Kontraktionsvorganges wird bei bekannten Herzschrittmachern zur Ratenadaption herangezogen, wobei zur Ratenberechnung ein auf klinischen Erfahrungen und entsprechenden Bewertungen basierender Algorithmus verwendet wird. Dieser nimmt auf den Unterschied zwischen dem Meß-Signalverlauf der intrakardialen Impedanz und einer Ruhe-Impedanz-Kurve als Referenz-Signalverlauf Bezug. Diese Ruhe-Kurve unterliegt längerfristigen Änderungen, die beispielsweise aufgrund von Medikamenteneinflüssen oder Veränderungen des Trainings-Zustandes des Patienten entstehen. Eine laufende Aktualisierung der Ruhe-Kurve wird daher im Stand der Technik vorgenommen.

Wie nun als Ausgangspunkt für die Entwicklung der vorliegenden Erfindung festgestellt wurde, ist die Kontraktionsdynamik bei verschiedenen Ereignissen und Ereignisfolgen, wie z. B. einerseits bei einer natürlichen, eigenstimulierten Kontraktion des Herzens und andererseits einer vom Herzschrittmacher erzeugten, atrialen Stimulation verschieden. Aus diesem Grund konnte bisher lediglich für ein ganz bestimmtes Trigger-Ereignis - also beispielsweise eine atriale und ventrikuläre Stimulierung des Herzens durch den Herzschrittmacher - ausgehend von einer einzigen Ruhe-Kurve eine Herzratenadaption durchgeführt wurde. Dies ist in physiologischer Hinsicht ungenügend.

Davon ausgehend liegt der Erfindung die Aufgabe zugrunde, ein medizinisches Geräteimplantat hinsichtlich der Bestimmung eines variablen Steuer-Parameters so zu verbessern, daß unterschiedliche Ausgangssituationen und die sich daraus ergebenden Einflüsse auf die Parameterbestimmung kompensiert werden.

Diese Aufgabe wird durch ein Geräteimplantat gemäß Anspruch 1 gelöst.

Im folgenden wird die Erfindung anhand der beigefügten Zeichnungen in einem Ausführungsbeispiel näher erläutert, wobei auf die beiliegenden Zeichnungen bezug genommen wird. Es zeigen:

Fig. 1   ein Kurvenschaubild von Ruhe-Impedanz-Kurven für unterschiedliche Ereignis-Typ-Folgen,

Fig. 2   ein Kurvenschaubild einer Impedanz-Kurve unter Belastung des Patienten, und

Fig. 3 ein Kurvenschaubild mit einer Ruhe- und Belastungs-Impedanz-Kurve.

Das Verfahren ist auf einem ratenadaptiven Herzschrittmacher implementiert, dessen Mikroprozessor-gestützte Steuerung in technisch grundsätzlich bekannter Weise dieses Verfahren umsetzt. Zur Ratenadaption - also zur Bestimmung der für den Patienten möglichst optimalen Herzrate, wie sie im Stimulationsfalle vom Herzschrittmacher anzuwenden ist - wird dieser variable physiologische Steuer-Parameter nach dem im folgenden zu erörterten Verfahren bestimmt. Ausgegangen wird dabei von der Messung der intrakardialen Impedanz des Herzens zwischen der an der Spitze des rechtsseitigen Ventrikels verankerten Herzschrittmacher-Elektrode und dem Gehäuse des Herzschrittmachers. Es wird also unipolar gemessen, wobei bi-phasische Rechteck-Pulse einer Dauer von wenigen zehn Mikrosekunden und einer Folge-Frequenz von einigen zehn Hertz als Meßstrom eingesetzt werden. Dieser Meßstrom bedingt einen Spannungsabfall in dem zwischen der Spitzen-Elektrode und dem Herzschrittmachergehäuse liegenden Bereich, der von der Impedanz sowohl des Herzmuskelgewebes als auch des im Ventrikel befindlichen Blutes abhängt. Bei der Kontraktion des Herzens ändert sich das Verhältnis zwischen Blut- und Muskelgewebemenge, so daß die intrakardiale Impedanz signifikant für den dynamischen Verlauf der Kontraktion ist. Basis-Parameter für das vorliegende Verfahren ist also diese dynamische intrakardiale Impedanz des Herzens während einer Kontraktion, deren Verhalten wiederum signifikant für die zu steuernde Herzrate ist.

Die vorstehende Tatsache läßt sich daraus erklären, daß sich bei einer Belastung des Patienten einerseits die Herzrate erhöht, andererseits aber auch das Kontraktionsverhalten des Herzens geändert wird. Insofern kann durch die Messung des Kontraktionsverhaltens des Herzens auf die einzustellende Herzrate geschlossen werden. Dabei wird grundsätzlich von einer Ruhe-Impedanz-Kurve ausgegangen, die durch Mittelung über mehrere Kontraktionen erhältlich ist. Diese Ruhe-Kontraktions-Kurve stellt den Referenz-Signalverlauf dar, auf dessen Basis durch einen noch zu erläuternden Algorithmus die Herzrate bestimmt wird.

Wie nun dieser Erfindung zugrundeliegt, weist der Referenz-Signalverlauf von unterschiedlichen Folgen von Trigger-Ereignissen signifikante Unterschiede auf. Ein Trigger-Ereignis ist dabei in dem hier erörterten Ausführungsbeispiel die eigentliche Herzschrittmacher-Aktion, die verschiedenen Typ-Folgen zugeordnet werden kann. So findet bei einem DDD-Herzschrittmacher eine atriale und ventrikuläre Abtastung der Herzaktion und eine atriale sowie ventrikuläre Stimulation im Bedarfsfall statt. Diese Ereignisse werden in der rechten Herzseite erfaßt bzw. initiiert. Werden sie auf die linke Herzseite ausgedehnt, nimmt entsprechend die Anzahl der unterschiedlich möglichen Ereignisse zu.

Typische Herzschrittmacher-Aktionen sind jeweils Ereignisfolgen des Typs "atriale Stimulation/ventrikuläre Stimulation" (abgekürzt mit Ap/Vp), atriale Stimulation/Erfassen einer ventrikulären Eigenaktion des Herzens" (Ap/Vs), "Erfassen einer atrialen Eigenaktion des Herzens/ventrikuläre Stimulation" (As/Vp) oder "Erfassen einer atrialen Eigenaktion des Herzens/ Erfassen einer ventrikulären Eigenaktion des Herzens" (As/Vs). Bei den vorstehenden Abkürzungen bedeuten also A" ein atriales Ereignis, "V" ein ventrikuläres Ereignis, p" (= pace) eine Stimulation des Herzens durch den Herzschrittmacher und s" (= sense) die Erfassung einer durch Eigenaktion des Herzens erfolgten, also praktisch natürlichen Kontraktion des Herzens durch den Herzschrittmacher. Es können im übrigen auch einzelne Herzschrittmacher-Aktionen des Typ Ap, As, Vp oder Vs als Trigger-Ereignis herangezogen werden.

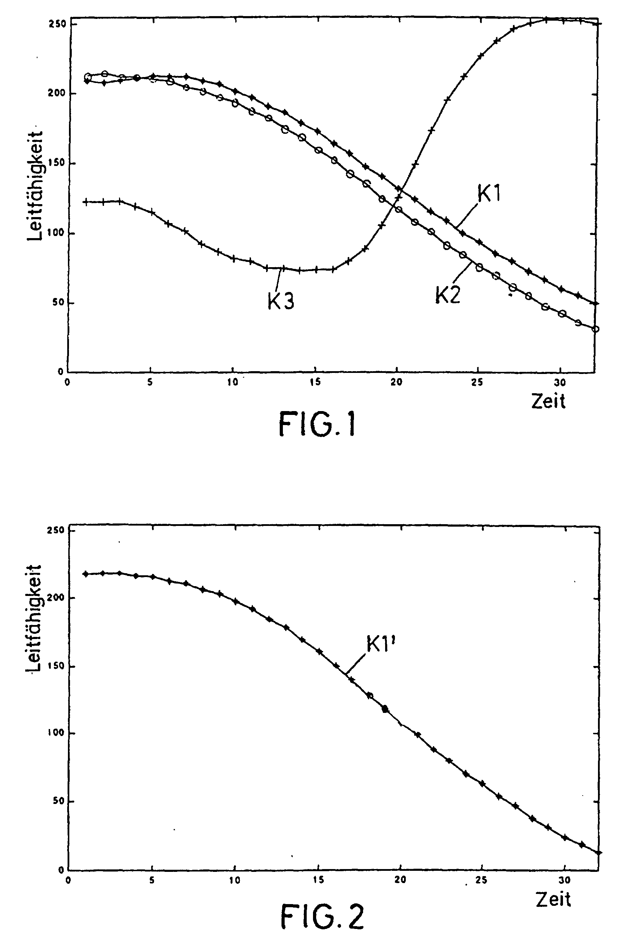

Bei der Entwicklung der vorliegenden Erfindung wurde nun festgestellt, daß die Ruhe-Impedanz-Kurven bei den vorstehenden unterschiedlichen Ereignis-Typ-Folgen Ap/Vp, Ap/Vs, As/Vp und As/Vs unterschiedlich sind. Dies wird aus Fig. 1 deutlich:

In dieser Zeichnung sind drei Meßkurven K1, K2 und K3 in einem Zeit-Leitfähigkeit-Diagramm aufgetragen, wobei auf den Achsen willkürliche Einheiten verwendet sind. Die Leitfähigkeit stellt qualitativ den Kehrwert der eigentlichen interessierenden Impedanz des Herzens dar.

Die Meßkurven K1, K2 und K3 wurden durch Mittelung mehrere Herzschrittmacher-Aktionen ermittelt. Die Meßkurve K1 stellt dabei die Ruhe-Kurve für die Ereignisfolge Ap/Vs dar. Die Meßkurve K2 gibt die Ereignisfolge As/Vs wieder und die Meßkurve K3 bezieht sich auf die Folge As/Vp. Die Meßkurve K3 macht deutlich, daß die dynamische Kontraktion des Herzens stark von der jeweiligen Herz- bzw. Herzschrittmacher-Aktion abhängt. Die Kurve K3 gibt - im Gegensatz zu den Kurven K1 und K2 - eine Ereignisfolge wieder, bei der ventrikulär durch den Herzschrittmacher stimuliert wurde (Ereignis-Typ Vp). Dabei läuft die Erregungsausbreitung im Vergleich zur Erregungsausbreitung bei einer spontanen Kontraktion in entgegengesetzte Richtung, was sich durch den drastisch veränderten Verlauf der Meßkurve K3 gegenüber den Meßkurven K1 und K2 ausdrückt.

Aus dem Vorstehenden wird die besondere Relevanz des Verfahrensschrittes deutlich, einen bestimmten Ereignis-Typ des Trigger-Ereignisses, das im Herzschrittmacher stattfindet, festzustellen und in Abhängigkeit des festgestellten Ereignis-Typs einen Referenz-Signalverlauf auszuwählen. Hat der Herzschrittmacher eben die Ereignisfolge As/Vp (Meßkurve K3) festgestellt, muß er diese Referenz-Kurve für die weitere Bestimmung der Herzrate zugrundelegen. Wurde die Ereignisfolge Ap/Vs festgestellt, ist eine Kurve des Typs K1 zu verwenden.

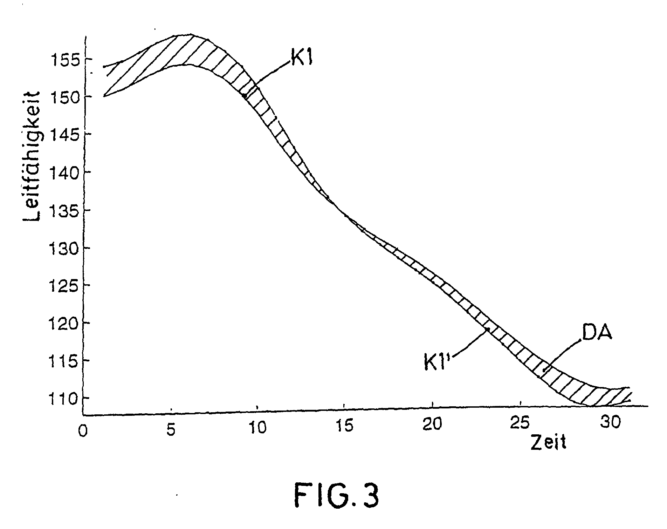

Figur 2 zeigt nun analog Figur 1 einen Meß-Signalverlauf unter Belastung eines Patienten, wobei als Ereignis-Typ die Ereignisfolge Ap/Vs, also "atriale Stimulation/Erfassen einer ventrikulären Eigenaktion des Herzens" vom Herzschrittmacher festgestellt wurde. Insoweit ist bei dem Verfahren also der Referenz-Signalverlauf gemäß Kurve K1 in Figur 1 heranzuziehen und der Meß-Signalverlauf gemäß K1' in Figur 2 mit diesem Referenz-Signalverlauf zu vergleichen. Daraus wird ein Vergleichswert ermittelt, der durch die absolute Differenzfläche zwischen beiden Kurven definiert ist. Dies ist anhand des Kurvenschaubilds gemäß Figur 3 zu erläutern, wo in qualitativ übereinstimmender Weise die beiden Signalverläufe K1 und K1' nochmals aufgetragen sind. Es wird deutlich, daß unter Belastung (Kurve K1') eine Kontraktion mit größerer Amplitude stattfindet, da der Maximal- und Minimalwert der Kurve K1' jeweils über bzw. unter der Ruhe-Impedanz-Kurve K1 liegen. Die absolute Differenzfläche DA ist in Figur 3 schraffiert dargestellt. In praxi wird sie dadurch in Näherung ermittelt, daß entlang der Signalverläufe K1, K1' an acht diskret ausgewählten Vergleichspunkten auf der Zeitachse eine Differenzbildung zwischen den jeweiligen Werten der Kurven K1 und K1' vorgenommen, die Absolutbeträge der Unterschiede aufaddiert und durch die Anzahl der Meßpunkte geteilt wird. Dies läßt sich durch die folgende Auswertungsformel darstellen:

$$DA = 1/8 \cdot \sum_{i=1}^{8} \left| K1(i) - K1'(i) \right|$$

Dieser jeweils ermittelte Vergleichswert DA geht nach einem im Herzschrittmacher-Steuerprogramm vorgegebenen Algorithmus in die Bestimmung des Steuer-Parameters - im vorliegenden Falle also der jeweils einzustellen Herzrate - ein.

Ein solche Algorithmus kann beispielsweise durch die folgende Beziehung realisiert sein:

$$HR = BR + rf \cdot DA$$

wobei bedeuten:

HR : Herzrate
BR : Basisrate
rf : Response-Faktor
DA : Differenzfläche

Aus der vorstehenden Beziehung wird also deutlich, daß zur Bestimmung des Steuer-Parameters Herzrate HR" die Differenzfläche DA mit einem Reponse-Faktor rf skaliert und die skalierte Differenzfläche als Summand einer Basis-Herzrate BR hinzu addiert wird. Die Basis-Herzrate stellt dabei einen physiologisch vernünftigen Wert für die Herzrate des Patienten in dessen Ruhezustand dar. Ausgehend von diesem wird die Herzrate bei Belastung erhöht.

Wie diagnostische Erkenntnisse gezeigt haben, sind die Kalibrationsfaktoren, wie die Basisrate BR und der Response-Faktor rf, genauso wie die eigentlichen Referenz-Signalverläufe K1, K2, K3 nicht statisch, sondern können sich mit unterschiedlichen Zeitkonstanten ändern. Insoweit ist es von Vorteil, wenn die an sich einschlägig bekannten Autokalibrations-Routinen in gängigen Herzschrittmachern nach vorgegebenen Zeiten alle Betriebszustände durchlaufen, um die Parameter zu aktualisieren. Insoweit werden nach festgelegten Perioden die relevanten Trigger-Ereignisse zwangsweise angesteuert. Es wird also beispielsweise auch für den Fall, daß das vom Herzschrittmacher gestützte Herz laufend eine eigeninitiierte Kontraktion des Atriums und Ventrikels bringt, durch eine entsprechende Aktivierung des Herzschrittmachers eine Stimulation des Atriums, des Ventrikels und beider Herzteile provoziert. Es werden also ausgehend von der Ereignis-Typ-Folge As/Vs die Ereignis-Folgen Ap/Vs, As/Vp und Ap/Vp zwangsweise angesteuert. Bei Auftreten dieser Ereignisse können dann die jeweiligen Referenz-Signalverläufe gespeichert werden und die bei Herzschrittmachern geläufigen Autokalibrations-Routinen ablaufen.

Es versteht sich ferner, daß die aus der Herzschrittmachertechnik bekannten Maßnahmen zur Herzraten-Stabilisierung, wie die sogenannte AV-Hysterese und die atriale Über-Stimulation (sogenannte "AV-Scans") auch im Zusammenhang mit dem Verfahren eingesetzt werden können.

## Patentansprüche

1. Medizinisches Geräteimplantat, insbesondere ratenadaptiver Herzschrittmacher, das eine Mikroprozessor-gestützte Steuerung aufweist in der ein Verfahren zur Bestimmung eines variablen, insbesondere physiologischen Steuer-Parameters eines medizinischen Geräteimplantates, insbesondere zur Herzratenadaption eines Herzschrittmachers, mit folgenden Verfahrensschritten implementiert ist:

   - Messen eines für den Steuer-Parameter signifikanten physiologischen Basis-Parameters nach Auftreten eines Trigger-Ereignisses,
   - Ermitteln eines Meß-Signalverlaufes für den Basis-Parameter aus dem vorstehenden Meßvorgang,
   - Feststellen eines bestimmten Ereignistyps des Trigger-Ereignisses,
   - Auswahl eines Referenz-Signalverlaufes in Abhängigkeit des festgestellten Ereignistyps,

- Vergleichen des Meß-Signalverlaufes mit dem ausgewählten Referenz-Signalverlauf,
- Bestimmung eines für den Unterschied zwischen Meß- und Referenz-Signalverlauf repräsentativen Vergleichswertes, und
- Bestimmung des Steuer-Parameters aus dem Vergleichswert nach einem vorgegebenen Rechen-Algorithmus, wobei

das Trigger-Ereignis eine Herzschrittmacher-Aktion oder eine Folge von Herzschrittmacher-Aktionen ist und die festgestellten Ereignistypen jeweils Ereignisfolgen des Typs atriale Stimulation/ventrikuläre Stimulation atriale Stimulation/Erfassen einer ventrikulären Eigenaktion des Herzens, Erfassen einer atrialen Eigenaktion des Herzens/ventrikuläre Stimulation oder Erfassen einer atrialen Eigenaktion des Herzens/Erfassen einer ventrikulären Eigenaktion des Herzens sind.

2. Geräteimplantat nach Anspruch 1, **dadurch gekennzeichnet, daß** der Basis-Parameter die dynamische intrakardiale Impedanz des Herzens während einer Kontraktion ist.

3. Geräteimplantat nach Anspruch 1 oder 2, **dadurch gekennzeichnet, daß** die Herzschrittmacher-Aktionen jeweils Ereignisse des Typs atriale Stimulation Erfassen einer atrialen Eigenaktion des Herzens, ventrikuläre Stimulation oder Erfassen einer ventrikulären Eigenaktion des Herzens sind.

4. Geräteimplantat nach einem der Ansprüche 1 bis 3, **dadurch gekennzeichnet, daß** für unterschiedliche Ereignistypen unterschiedliche Ruhe-Impedanz-Kurven als Referenz-Signalverlauf ausgewählt werden.

5. Geräteimplantat nach Anspruch 4, **dadurch gekennzeichnet, daß** der Vergleichswert für den Unterschied zwischen Meß- und Referenz-Signalverlauf die absolute Differenzfläche zwischen dem nach dem Ereignis-Typ ausgewählten Referenz-Signalverlauf und dem jeweiligen Meß-Signalverlauf ist.

6. Geräteimplantat nach Anspruch 5, **dadurch gekennzeichnet, daß** Referenz- und Meß-Signalverlauf jeweils diskret an ausgewählten Vergleichspunkten einer Differenzbildung unterworfen werden.

7. Geräteimplantat nach Anspruch 5 oder 6, **dadurch gekennzeichnet, daß** die Differenzfläche mit einem Response-Faktor skaliert und die skalierte Differenzfläche zur Herzratenadaption eines Herzschrittmachers als Summand einer Basis-Herzrate hinzuaddiert wird.

8. Geräteimplantat nach einem der Ansprüche 1 bis 7, **dadurch gekennzeichnet, daß** zur Anpassung der für die Bestimmung der jeweiligen Vergleichswerte und Steuer-Parameter anzuwendenden Kalibrationsfaktoren nach festgelegten Perioden die relevanten Trigger-Ereignisse zwangsweise angesteuert werden.

9. Geräteimplantat nach einem der Ansprüche 1 bis 8, **dadurch gekennzeichnet, daß** die Meß-Signalverläufe durch Mittelwert-Bildung über mehrere Meßvorgänge ermittelt werden.

**Claims**

1. Implantable medical device, particularly a rate-adaptive pacemaker, having a microprocessor-aided control that implements a method for determining a variable, especially physiological, control parameter for an implantable medical device, particularly for a heart rate adaptation in a pacemaker, comprising the following procedural steps:

- measuring a physiological base parameter that is significant for the control parameter, after a trigger event has occurred,
- determining a measuring signal curve for the base parameter from the above measuring process,
- determining a certain event type of the trigger event,
- selecting a reference signal curve in dependence on the determined event type,
- comparing the measuring signal curve to the selected reference signal curve,
- determining a comparison value that is representative for the difference between the measuring signal curve and the reference signal curve, and
- determining the control parameter from the comparison value according to a predefined calculation algorithm, wherein the trigger event is a pacemaker action or a succession of pacemaker actions and the event types observed are event sequences of the respective types atrial stimulation/ventricular stimulation, atrial stimulation/sensing of a natural ventricular action of the heart, sensing of a natural atrial action of the heart/ventricular stimulation, or sensing of a natural atrial action of the heart/sensing of a natural ventricular action of the heart.

2. An implantable medical device according to claim 1, **characterized in that** the base parameter is the dynamic intracardiac impedance of the heart during a contraction.

**3.** An implantable medical device according to claim 1 or 2, **characterized in that** the pacemaker actions are events of the respective types atrial stimulation, sensing of a natural atrial action of the heart, ventricular stimulation, or sensing of a natural ventricular action of the heart.

**4.** An implantable medical device according to one of the claims 1 through 3, **characterized in that** for different event types, different atrest impedance curves are selected as the reference signal curve.

**5.** An implantable medical device according to claim 4, **characterized in that** the comparison value for the difference between the measuring signal curve and the reference signal curve is the absolute differential area between the reference signal curve that was selected based on the event type and the respective measuring signal curve.

**6.** An implantable medical device according to claim 5, **characterized in that** the reference signal curve and the measuring signal curve are subjected to a subtraction in discretely selected comparison points.

**7.** An implantable medical device according to claim 5 or 6, **characterized in that** the differential area is scaled with a response factor and the scaled differential area is added as an addend to a base heart rate to adapt the heart rate of a pacemaker.

**8.** An implantable medical device according to one of the claims 1 through 7, **characterized in that** to adapt the calibration factors to be applied in the determination of the respective comparison values and control parameters, the relevant trigger events are forced after predetermined time periods.

**9.** An implantable medical device according to one of the claims 1 through 8, **characterized in that** the measuring signal curves are determined by averaging across multiple measuring processes.

## Revendications

**1.** Implant médical, plus particulièrement un stimulateur cardiaque à rythme adaptable qui comprend une commande assistée par un microprocesseur, dans laquelle se déroule un procédé de détermination d'un paramètre de commande variable, notamment physiologique, d'un implant médical, plus particulièrement pour l'adaptation du rythme cardiaque d'un stimulateur cardiaque, avec les étapes de procédé suivantes :

- Mesure d'un paramètre de base physiologique significatif pour le paramètre de commande, après l'apparition d'un événement déclencheur,

- Détermination d'une courbe de signal de mesure pour le paramètre de base à partir du processus de mesure précédent,

- Détermination du type d'événement de l'événement déclencheur,

- Sélection d'une courbe de signal de référence en fonction du type d'événement déterminé,

- Comparaison de la courbe du signal de mesure avec la courbe de signal de référence,

- Détermination d'une valeur de comparaison représentative de la différence entre la courbe de signal de mesure et la courbe de signal de référence, et

- Détermination du paramètre de commande à partir de la valeur de comparaison selon un algorithme de calcul prédéterminé, moyennant quoi

l'événement déclencheur est une action sur le stimulateur cardiaque ou une suites d'actions sur le stimulateur cardiaque et les évènements déterminés sont des suites d'évènements du type stimulation atriale/stimulation ventriculaire, stimulation atriale/détermination d'une action ventriculaire propre du coeur, détermination d'une action atriale propre du coeur/stimulation ventriculaire, détermination d'une action atriale propre du coeur/détermination d'une action ventriculaire propre du coeur.

**2.** Implant médical selon la revendication 1, **caractérisé en ce que** le paramètre de base est l'impédance dynamique intracardiaque du coeur pendant une contraction.

**3.** Implant médical selon la revendication 1 ou 2, **caractérisé en ce que** les actions du stimulateur cardiaque sont des évènements du type stimulation atriale, détermination d'une action atriale propre du coeur, une stimulation ventriculaire ou la détermination d'une action ventriculaire propre du coeur.

**4.** Implant médical selon l'une des revendications 1 à 3, **caractérisé en ce que**, pour différents types d'évènements, différentes courbes d'impédance au repos sont choisies comme courbes de signal de référence.

**5.** Implant médical selon la revendication 4, **caractérisé en ce que** la valeur de comparaison pour la différence entre la courbe de signal de mesure et la courbe de signal de référence est la surface différentielle absolue entre la courbe de signal de référence choisie selon le type d'événement et la courbe de signal de mesure correspondante.

**6.** Implant médical selon la revendication 5, **caractérisé en ce que** la courbe de signal de référence et la courbe de signal de mesure sont soumises, de manière discrète, au niveau de points de comparaison, à la détermination d'une différence.

**7.** Implant médical selon la revendication 5 ou 6, **caractérisé en ce que** la surface différentielle est cadrée avec un facteur de réponse et la surface différentielle cadrée est ajoutée à l'adaptation du rythme cardiaque d'un stimulateur cardiaque en tant qu'opérande de la somme d'un rythme cardiaque de base.

**8.** Implant médical selon l'une des revendications 1 à 7, **caractérisé en ce que**, pour l'adaptation des facteurs de calibrage à utiliser pour la détermination des valeurs de comparaison et paramètres de commande, les évènements déclencheurs sont provoqués après des périodes déterminées.

**9.** Implant médical selon l'une des revendications 1 à 8, **caractérisé en ce que** les courbes de signal de mesure sont déterminées par la formation d'une valeur moyenne par l'intermédiaire de plusieurs processus de mesure.

FIG.1

FIG.2

FIG.3